# EUROPÄISCHE PATENTSCHRIFT

(11) **EP 1 195 143 B1**
(45) Veröffentlichungstag und Bekanntmachung des Hinweises auf die Patenterteilung: **07.12.2005**
(21) Anmeldenummer: 01118469.4
(22) Anmeldetag: 01.08.2001
(51) Int. Cl.: A61B 17/72, A61B 17/17

(54) **Knochennagel**
Intramedullary nail
Clou intramédullaire

(30) Priorität: 12.09.2000 DE 20015775 U
(43) Veröffentlichungstag der Anmeldung: 10.04.2002
(73) Patentinhaber: Stryker Trauma GmbH, 24232 Schönkirchen (DE)
(72) Erfinder: Simon, Bernd, 24106 Kiel (DE); Füllgraf, René, Dr., 24118 Kiel (DE)
(74) Vertreter: Hauck, Graalfs, Wehnert, Döring, Siemons

(56) Entgegenhaltungen:
- EP-A- 0 523 905
- WO-A-97/13467
- US-A- 5 411 503
- US-A- 6 074 394

## Beschreibung

Die Erfindung betrifft einen Knochennagel, nach dem Oberbegriff des Anspruchs 1.

Zur Versorgung von Knochenfrakturen in Röhrenknochen ist bekannt, sogenannte Verriegelungsnägel zu verwenden. Sie weisen Querbohrungen auf, durch die Knochenschrauben hindurchgeführt werden, um die Knochenfragmente am Knochennagel zu halten und diese gegen Verschiebung und Torsion zu sichern. Bevor die Knochenschrauben eingeschraubt werden können, müssen Löcher in die Kortikalis gebohrt werden. Diese Löcher müssen relativ genau auf die Querbohrungen des Knochennagels ausgerichtet sein.

Es sind zahlreiche Verfahren und Vorrichtungen bekannt geworden, die Stelle aufzufinden, an der eine Bohrung für die Knochenschraube zu setzen ist. Die meisten Zielvorrichtungen arbeiten nach dem Prinzip der Durchleuchtung. Der Operateur kann auf dem Monitor die Lage der Querbohrungen im Knochennagel innerhalb des Knochens ermitteln und den Bohrer entsprechend ansetzen. Es sind jedoch auch mechanische Zielgeräte bekannt, die auf das freie Ende des Knochennagels aufsetzbar sind. Der Abstand der Querbohrung vom Ende des Knochennagels ist bekannt. Auch die Drehlage der Querbohrung kann durch eine entsprechende Markierung am Ende des Knochennagels angegeben sein. Mit Hilfe einer geeigneten Zielhülse kann dann über die Zielgeräte die Kortikalis gebohrt werden. Bei dem mechanischen Zielgerät ist gegenüber dem Durchleuchtungsverfahren der Patient und sein Operateur keiner Röntgenstrahlung ausgesetzt. Nachteilig an dem mechanischen Zielgerät ist jedoch, dass eine Verwindung des Nagels beim Einschlagen zu Abweichungen in der Lage der Querbohrungen führt, die durch das Zielgerät nicht erfasst werden können.

Aus US -A- 5,411,503 ist ein Instrument zum Zielen für Verriegelungsschrauben in Markknochen bekannt geworden. Bei dem Verfahren wird ein Spieß in den hohlen Knochennagel eingeführt. Der Spieß ist an seinem distalen Ende mit zwei Schwingkreisen versehen. An dem proximalen Ende weist der Spieß ein Anschlagelement auf. Zur Positionierung eines Zielgeräts wird der Spieß vollständig in den eingetriebenen Knochennagel eingesetzt, wobei das Anschlagelement an dem proximalen Ende des Nagels aufliegt. Die beiden Schwingkreise am distalen Ende des Spießes werden angeregt und das von ihnen ausgesendete elektromagnetische Feld wird durch entsprechende Sensoren an einem Zielgerät erfasst. Das Zielgerät wird in dem Feld der Schwingkreise derart ausgerichtet, dass ein mit diesem verbundener Führungskanal für den Bohrer entsprechend der Querbohrung an dem Knochennagel ausgerichtet ist. Bei dieser Vorrichtung ist nachteilig, dass eine während des Eintreibens des Nagels auftretende Verwindung oder Stauchung des Knochennagels, ebenso wie bei den oben beschriebenen mechanischen Zielgeräten, nicht berücksichtigt werden kann.

Aus der US 5,584,838 ist ein Knochennagel bekannt geworden, in dessen hohlen Schaft ein Element mit einem einzelnen Schwingkreis eingesetzt ist. Der Nagel wird mit eingesetztem Element in den Knochen eingetrieben, so dass die vorstehenden Nachteile einer Verwindung des Nagels nicht auftreten können. Nachteilig an einem derart ausgebildeten Knochennagel ist, dass durch die Verwendung nur eines Schwingkreises in dem Knochennagel ein vergleichsweise aufwendiges Zielgerät verwendet werden muss, um den Bohrer auszurichten. Ferner erweist es sich als nachteilig, dass der Schwingkreis genau in dem Kanal der Querbohrung angeordnet ist, damit die Lage der Querbohrung korrekt erfasst werden kann. Nachdem der Kanal der Querbohrung erfasst wurde, wird das eingesetzte Element entfernt.

Aus EP-A-0 523 905 ist bekannt, in einen Knochennagel einen Einsatz einzuführen, nachdem der Knochennagel eingetrieben wurde. Der Einsatz enthält eine Sendereinrichtung, die bei Erregung Signale abstrahlt zur Detektierung der Lage einer Querbohrung des Knochenmarkes.

Der Erfindung liegt die Aufgabe zugrunde einen Knochennagel bereitzustellen, bei dem die Position seiner Querbohrungen im eingetriebenen Zustand mit einfachen Mitteln zuverlässig festgestellt werden kann.

Erfindungsgemäß wird die Aufgabe durch einen Knochennagel mit den Merkmalen des Anspruchs 1 gelöst.

Der Knochennagel nach der Erfindung weist einen hohlen Schaft auf, der mit mindestens einer Querbohrung zur Aufnahme einer Knochenschraube oder dergleichen versehen ist, wie an sich bekannt. Im Bereich der Querbohrung jedoch im Abstand dazu ist die Wandung des Schaftes mit mindestens einer Ausnehmung oder Durchbrechung versehen, die eine Sendeeinrichtung dauerhaft aufnimmt. Sie hat einen vorbestimmten Abstand und eine vorbestimmte Ausrichtung relativ zur Querbohrung und strahlt bei Erregung Signale ab. Mit Hilfe eines Zielgeräts, das von dem Operateur außerhalb des Nagels geführt wird, können diese Signale erfasst werden. Anhand der Lage und der Orientierung der abgestrahlten Strahlung, kann in axialer Richtung und Umfangsrichtung die Position der Querbohrung bestimmt werden.

Nach einer Ausgestaltung der Erfindung weist der Schaft einen darin angeordneten Einsatz auf, der in seiner Wandung mindestens eine Ausnehmung oder Durchbrechung aufweist, welche die Sendeeinrichtung aufnimmt. Bevorzugt ist der Einsatz hülsenförmig ausgebildet. Der Einsatz ist in dem Schaft des Knochennagels ausgerichtet, wodurch eine genaue Bestimmung der Bohrung auch im eingeschlagenen Zustand des Nagels möglich ist. Durch die bevorzugte Verwendung von mindestens zwei Sendeeinrichtungen in dem Einsatz können vergleichsweise einfach aufgebaute Zielgeräte verwendet werden. Bei der bevorzugten Verwendung eines hülsenförmigen Einsatzes mit einem Durchgangskanal und der Anordnung der Sendeeinrichtungen in der Einsatzwandung verbleibt ein freier Durchgangskanal in dem Schaft, der beispielsweise ein Eintreiben des Knochennagels entlang eines Führungsspießes ermöglicht. Wie erwähnt, verbleiben bei dem erfindungsgemäßen Knochennagel die Sendeeinrichtungen dauerhaft in dem Knochennagel.

Um eine korrekte Ausrichtung des Einsatzes in axialer Richtung auch nach dem Einschlagen des Nagels sicherzustellen, ist nach einer Ausgestaltung der Erfindung in dem Schaft ein nach innen vorstehender Absatz oder Vorsprung geformt, an dem der in den Schaft eingesetzte Einsatz in axialer Richtung anliegt. Dabei schließt der Einsatz bündig mit der Innenwandung des Schaftes ab, so dass eine im wesentlichen durchgehend ebene Innenwandung entsteht. Bevorzugt ist der Absatz an dem proximalen Ende des Einsatzes vorgesehen, so dass dieser sich an dem Absatz während des Eintreibvorgangs abstützt.

Für eine Ausrichtung des Einsatzes in radialer Richtung ist der Einsatz nach einer weiteren Ausgestaltung der Erfindung mit quer zur Längsrichtung verlaufenden Bohrungen versehen, die entsprechend den Querbohrungen im Schaft ausgerichtet sind. Die Bohrungen in dem Einsatz ermöglichen es, den Einsatz im eingesetzten Zustand in dem Schaft entsprechend den Bohrungen auszurichten, wodurch die Sendeeinrichtungen in dem Einsatz ebenfalls abhängig von den Bohrungen ausgerichtet werden.

Bevorzugt weist der Einsatz zusätzlich ein hülsenförmiges Kontaktelement auf, das eine oder mehrere Ausnehmungen oder Durchbrechungen für die Sendeeinrichtungen in dem Einsatz abdeckt und das mit der Sendeeinrichtung elektrisch leitend verbunden und gegenüber dem Einsatz isoliert ist. Ein solches Kontaktelement vergrößert die Kontaktfläche der Sendeeinrichtung. Bevorzugt steht die Sendeeinrichtung zusätzlich mit der Wand des Knochennagels als zweitem Kontakt in Verbindung. Die in den Einsatz eingesetzte Sendeeinrichtung ist hierbei ebenfalls gegenüber dem Einsatz elektrisch isoliert. Bei dieser Ausgestaltung der Erfindung fließt ein Strom über das Kontaktelement, durch die Sendeeinrichtung und in die Wand des Knochennagels.

Abhängig von der Ausgestaltung des Schaftes können hierbei die Ausnehmungen in der Schaftwandung wahlweise von innen oder von außen zugänglich sein.

Für eine zuverlässige Ortung der von den Sendeeinrichtungen abgestrahlten Signale erweist es sich als vorteilhaft, die Sendeeinrichtungen im Einsatz oder in der Schaftwandung jeweils paarweise in axialer Richtung zueinander versetzt anzuordnen. Hierdurch entstehen zwei parallele Abstrahlungscharakteristiken, die eine zuverlässige Ausrichtung eines Zielgeräts ermöglichen.

In einer zweckmäßigen Ausgestaltung der Erfindung handelt es sich bei der Sendeeinrichtung um ein aktives oder passives Bauelement, bevorzugt in Form einer Tablette, das auf empfangene oder angelegte Anregungssignale ansprechend ein Sendesignal ausstrahlt. Bevorzugt handelt es sich bei dem Bauelement um einen passiven Schwingkreis. Auch kann als Bauelement ein Transponder vorgesehen sein. Die Verwendung von passiven Sendeeinrichtungen ist insbesondere im Hinblick auf deren Verbleib in dem Knochennagel vorteilhaft, da keine Energieversorgung für diese in dem Knochennagel vorgesehen werden muss. Die Sendeeinrichtung kann die Form einer Tablette aufweisen, deren Stirnfläche an den Innendurchmesser angepasst konkav gewölbt ist.

Die elektrischen Sendesignale können an die innere Kontaktfläche der Sendeeinrichtung über eine stabförmige Vorrichtung angelegt werden, wobei die stabförmige Vorrichtung einen elektrisch isolierten Schaft besitzt, dessen Außendurchmesser kleiner als der Innendurchmesser des Schafts des hohlen Knochennagel ist, und die mit einer elektrisch leitenden Spitze verbunden ist. Diese Vorrichtung gestattet es, mit Hilfe der elektrisch leitenden Spitze an die Sendeeinrichtungen ein Anregungssignal anzulegen, das ein Sendesignal auslöst. Insbesondere bei der Verwendung von Schwingkreisen erweist sich dies als vorteilhaft, da Anregungssignale mit einer Frequenz an die Sendeeinrichtung angelegt werden können, die der Resonanzfrequenz der Sendeeinrichtung entsprechen. Bevorzugt weisen Paare von Sendeeinrichtungen unterschiedliche Resonanzfrequenzen auf. Bei der Verwendung von Transpondern ist es auch möglich, deren Anregungssignale von innerhalb oder außerhalb des Schaftes zu senden.

Mit Bezug auf die beiliegenden Zeichnungen wird nachfolgend ein bevorzugtes Ausführungsbeispiel näher beschrieben. Es zeigt:
- Figur 1: eine auseinander gezogene Darstellung einer Spitze eines erfindungsgemäßen Knochennagels im Längsschnitt,
- Figur 2: eine perspektivische Ansicht einer zusammengesetzten Nagelspitze mit Einsatz im Längsschnitt und
- Figur 3: einen schematischen Querschnitt durch einen erfindungsgemäßen Knochennagel im Bereich einer Querbohrung.

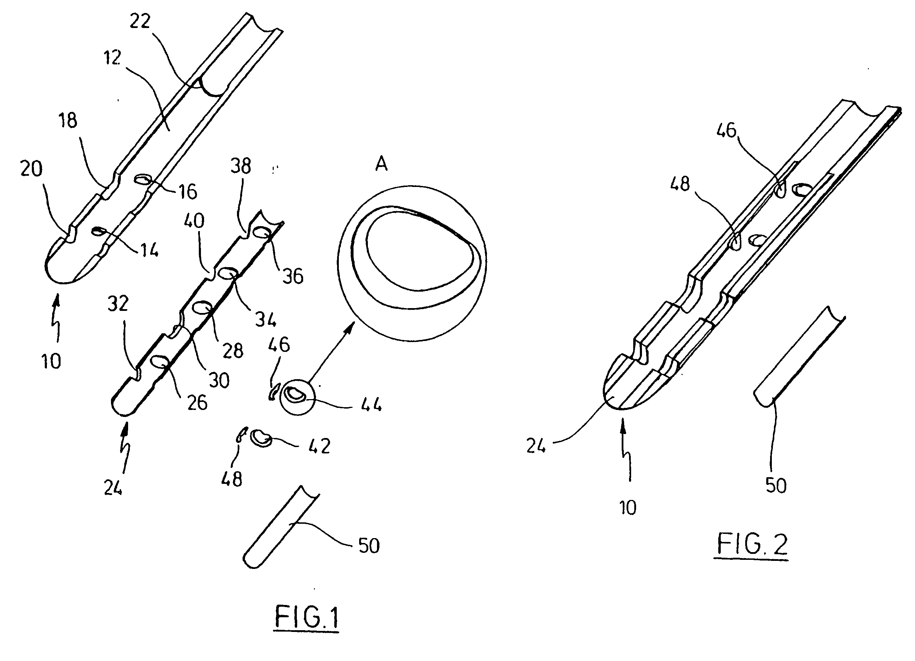

Figur 1 zeigt die Spitze eines Knochennagels 10 im Längsschnitt. Der Nagel besitzt einen im Querschnitt kreisförmigen Kanal 12, der am distalen Ende des Nagels 10 offen ist. In der Wandung des Nagels 10 sind Bohrungen 14 und 16 vorgesehen. In dem dargestellten Längsschnitt sind zwei weitere Bohrungen 18, 20 geschnitten dargestellt, die versetzt zu den Bohrungen 14 und 16 in dem Schaft angeordnet sind. In dem Kanal 12 ist ein Absatz 22 ausgebildet.

Der als Hülse 24 ausgebildete Einsatz ist ebenfalls im Längsschnitt dargestellt. Die Hülse ist hohl und besitzt einen Außendurchmesser, der dem Durchmesser des Kanals 12 entspricht. Die Hülse 24 besitzt zwei Bohrungen 26 und 28, die im eingesetzten Zustand der Hülse mit den Bohrungen 14 und 16 korrespondieren. Zu den quer dazu verlaufenden Bohrungen 18 und 20 des Knochennagels sind entsprechende Bohrungen 30 und 32 in der Hülse 24 vorgesehen. Zusätzlich zu den Bohrungen, die den Querbohrungen des Knochennagels entsprechen, sind in der Hülse Durchbrechungen 34 und 36 vorgesehen. Die den Durchbrechungen 34 und 36 zugeordneten Durchbrechungen 38 und 40 sind in dem dargestellten Längsschnitt angeschnitten.

In die Durchbrechungen 34 bis 40 werden die Sendeeinheiten 42 bis 48 eingesetzt. Die Sendeeinheit 44 ist in der Ansicht A vergrößert dargestellt. Sie besitzt eine im Wesentlichen runde Form, wobei die Grundflächen an die Wölbung des Kanals 12 in dem Nagel 10 angepasst ist. Die Dicke der Sendeeinheit entspricht hierbei der Dicke der Hülse im Bereich der Durchbrechung. Diese Gestaltung verleiht der Sendeeinrichtung ungefähr die Form einer Tablette. Abgedeckt werden die eingesetzten Sendeeinheiten durch das hülsenförmige Kontaktelement 50.

Figur 2 zeigt die Nagelspitze 10 mit eingesetzter Hülse 24. Die Sendeeinrichtungen 42 bis 48 sind ebenfalls in die Hülse 24 eingesetzt und werden durch das Kontaktelement 50 abgedeckt. Hülse 24 und Kontaktelement 50 liegen in axialer Richtung des Nagels an dem Absatz 22 an.

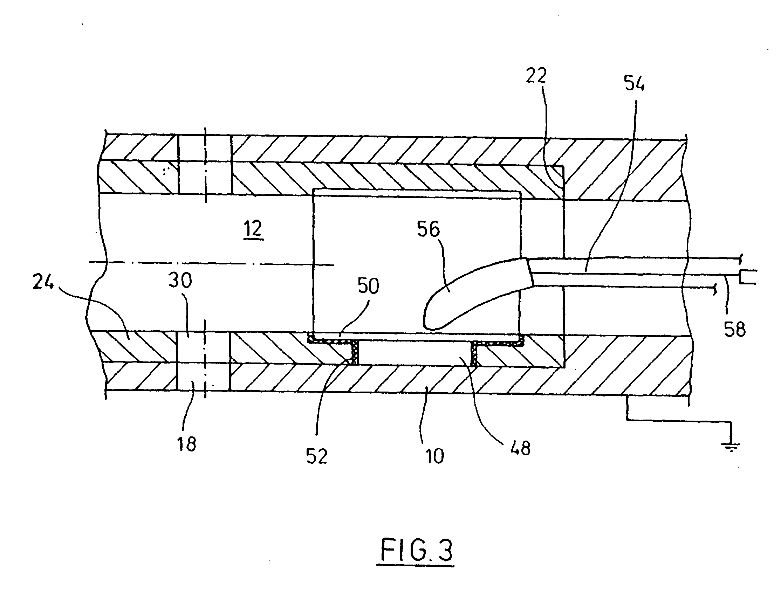

Figur 3 zeigt einen Querschnitt durch den Nagel 10 im Bereich der Querbohrung 30. In den Nagel 10 eingesetzt ist die Hülse 24 mit ihrer Bohrung 30. Die Hülse ist in der Nagelspitze derart ausgerichtet, dass die Bohrung 30 mit der Bohrung 18 der Nagelaußenwand zusammenfällt. Die Sendeeinheit 48 ist beabstandet von der Bohrung 30 in der Durchbrechung 40 der Hülse angeordnet. Die Sendeeinheit 48 steht an ihren Stirnseiten in elektrischem Kontakt mit der Wandung 10 des Nagels und dem eingesetzten Kontaktelement 50. Außerhalb der Kontaktbereiche ist der Sender 48 gegenüber der Hülse 24 durch einen Isolator 52 abgeschirmt.

In den Kanal 12 des Nagels eingeführt ist ein Spieß 54, dessen elektrisch leitende Spitze 56 über einen elektrischen Leiter 58 mit einer Signalquelle verbunden ist. An der Sendeeinheit 48 wird mit Hilfe der abgebogenen Spitze 56 ein elektrisches Anregungssignal angelegt, das bei der Sendeeinheit 48 das Senden von entsprechenden Signalen auslöst. Hierzu ist die Außenwandung des Nagels geerdet.

Alternativ zu der in Figur 3 dargestellten Ausführungsform ist es ebenfalls möglich, die Sendeeinheit 48 durch elektromagnetische Wellen zur Abstrahlung von Signalen anzuregen, wie es beispielsweise bei Transpondern möglich ist. Die Quelle zur Anregung eines Transponders kann ebenfalls über einen Spieß in den Schaft eingeführt werden. Alternativ ist es bei der Wahl von geeigneten Anregungsfrequenzen für die Anregungssignale möglich, die Sendeeinheiten auch von außen anzuregen. Die Hülse 24 verbleibt mit ihren Sendeeinheiten in dem Knochennagel, nachdem die Knochenschrauben eingesetzt wurden.

## Patentansprüche

1. Knochennagel mit einem hohlen Schaft, der mindestens eine Querbohrung (14 - 20) zur Aufnahme einer Knochenschraube aufweist und mindestens einer Sende-einrichtung (42 - 48), wobei die Sendeeinrichtung in einem vorbestimmten Abstand und in einer vorbestimten Ausrichtung relativ zur Querbohrung (14 - 20) bei Erregung Signale abstrahlt zur Detektierung der Lage der Querbohrung (14 - 20) aus den Signalen, **dadurch gekennzeichnet, daß** die Wandung des Schaftes mindestens eine Ausnehmung oder Durchbrechung aufweist, die die Sendeeinrichtung (42 - 48) dauerhaft innerhalb des Schaftes des Knochennagels (10) aufnimmt.

2. Knochennagel nach Anspruch 1, **dadurch gekennzeichnet, dass** ein Einsatz (24) im Schaft vorgesehen ist, der in seiner Wandung mindestens eine Ausnehmung oder Durchbrechung (34 - 40) aufweist, welche die Sendeeinrichtung (42 - 48) aufnimmt.

3. Knochennagel nach Anspruch 2, **dadurch gekennzeichnet, dass** der Einsatz (24) hülsenförmig mit einem in Längsrichtung des Nagels verlaufenden Durchgangskanal (12) ausgebildet ist.

4. Knochennagel nach Anspruch 2 oder 3, **dadurch gekennzeichnet, dass** der hohle Schaft eine Vertiefung mit einem nach innen vorspringenden Absatz oder Vorsprung (22) aufweist, wobei der in den Schaft eingesetzte Einsatz in axialer Richtung an dem Absatz oder Vorsprung anliegt und mit der Innenwandung des Schaftes bündig abschließt.

5. Knochennagel nach Anspruch 2 oder 3, **dadurch gekennzeichnet, dass** der Einsatz (24) quer zur Längsrichtung verlaufende Bohrungen (26 - 32) aufweist, die mit den Querbohrungen (16 - 20) im Schaft ausgerichtet sind.

6. Knochennagel nach einem der Ansprüche 2 oder 3, **dadurch gekennzeichnet, dass** ein hülsenförmiges Kontaktelement (50) innerhalb des Einsatzes (24) die Ausnehmung oder Durchbrechung abdeckt und mit der Sendeeinrichtung (42 - 48) elektrisch leitend verbunden und gegenüber dem Einsatz (24) elektrisch isoliert ist.

7. Knochennagel nach Anspruch 6, **dadurch gekennzeichnet, dass** zwischen der Sendeeinrichtung (48) und dem Einsatz (24) ein Isolator (52) angeordnet ist, der die Sendeeinrichtung (48) gegenüber dem Einsatz (24) isoliert und die Sendeeinrichtung (48) eine elektrisch leitende Verbindung zum Schaft bildet.

8. Knochennagel nach einem der Ansprüche 1 bis 7, **dadurch gekennzeichnet, dass** der Schaft einen hohlen Kanal (12) aufweist.

9. Knochennagel nach einem der Ansprüche 2 bis 8, **dadurch gekennzeichnet, dass** die Sendeeinrichtungen (42 - 48) jeweils paarweise, in axialer Richtung zueinander versetzt angeordnet sind.

10. Knochennagel nach einem der Ansprüche 2 bis 9, **dadurch gekennzeichnet, dass** die Sendeeinrichtung (42 - 48) die Form einer Tablette aufweist, deren eine Stirnfläche an den Innendurchmesser des Schaftes angepasst konkav gewölbt und deren Höhe im wesentlichen der Tiefe der Ausnehmung oder Durchbrechung (34 - 40) entspricht.

11. Knochennagel nach einem der Ansprüche 1 bis 10, **dadurch gekennzeichnet, dass** die Sendeeinrichtung ein passives oder aktives Bauelement aufweist, das auf empfangene oder angelegte Anregungssignale ansprechend ein Sendesignal ausstrahlt.

12. Knochennagel nach Anspruch 11, **dadurch gekennzeichnet, dass** das Bauelement als ein passiver Schwingkreis ausgebildet ist.

13. Knochennagel nach einem der Ansprüche 11 oder 12, **dadurch gekennzeichnet, dass** die Sendeeinrichtung zwei elektrische Kontakte zum Anlegen eines Anregungssignals mittels eines in den Schaft eingeführten Leiters aufweist.

14. Knochennagel nach Anspruch 13, **dadurch gekennzeichnet, dass** einer der Kontakte mit dem Schaft des Knochennagels in elektrisch leitender Verbindung steht.

## Claims

1. A bone nail with a hollow shank which has at least one cross-bore (14 - 20) to receive a bone screw, and at least one transmitting unit (42 - 48) wherein the transmitting unit is adapted to emit signals at a predetermined spacing from and in a predetermined alignment relative to the cross-bore (14 - 20), which signals can be detected in order to determine the position of the cross-bore (14-20), **characterized in that** the hollow shank exhibits at least one recess or through opening in its wall to accommodate the transmitting unit (42 - 28) permanently inside the hollow shank of the bone nail (10).

2. The bone nail according to claim 1, **characterized in that** an insert (24) is provided in the hollow shank with a recess or through opening (34 - 40) which receives the transmitting unit (42 - 48).

3. The bone nail according to claim 2, **characterized in that** the insert (24) is formed in a sleeve-like fashion with a. through passage (12) extending in the longitudinal direction of the nail.

4. The bone nail according to claim 2 or 3, **characterized in that** the hollow shank has a dimple including an inwardly projecting shoulder or protrusion (22) wherein the insert introduced into the shank bears on the shoulder or protrusion in an axial direction and finishes in a flush fashion with the inner wall of the shank.

5. The bone nail according to claim 2 or 3, **characterized in that** the insert (24) has bores (26 - 32) extending transversely to the longitudinal direction which are properly aligned according to the cross-bores (16 - 20) in the shank.

6. The bone nail according to any of the claims 2 or 3, **characterized in that** a sleeve-shaped contact member (50) within the insert (24) covers the recess or through opening and is connected to the transmitting unit (42 - 28) in an electrically conductive way and is insulated against the insert (24).

7. The bone nail according to claim 6, **characterized in that** an insulator (52) is disposed between the transmitting unit (48) and the insert (24) which insulates the transmitting unit (48) from the insert (24) and the transmitting unit (48) forms an electrically conductive connection between the contact member and the shank.

8. The bone nail according to any of the claims 1 to 7, **characterized in that** the shank exhibits a hollow channel (12).

9. The bone nail according to any of the claims 2 to 8, **characterized in that** the transmitting units (42 - 48) are disposed in pairs each and are offset from each other in an axial direction.

10. The bone nail according to any one of claims 2 to 9, **characterized in that** the transmitting unit (42 - 48) substantially is of the shape of a tablet one front-end face of which is concavely bulged, adapting itself to the inside diameter of the shank, and the height of which substantially corresponds to the depth of the recess or through opening (34 - 40).

11. The bone nail according to any one of claims 1 to 10, **characterized in that** the transmitting unit has a passive or active component which emits a transmit signal in response to excitation signals which are received or applied thereto.

12. The bone nail according to claim 11, **characterized in that** the component is formed as a passive oscillating circuit.

13. The bone nail according to any one of claims 11 or 12, **characterized in that** the transmitting unit has two electric contacts for applying an excitation signals by means of a conductor inserted in the nail.

14. The bone nail according to claim 13, **characterized in that** one of the contacts is in an electrically conductive connection with the shank of the bone nail.

## Revendications

1. Clou intramédullaire avec un fût creux, qui présente au moins un alésage transversal (14 - 20) pour loger une vis à os et au moins un équipement d'émission (42 - 48), l'équipement d'émission émettant, à une distance prédéfinie et dans un alignement prédéfini par rapport à l'alésage transversal (4 - 20), en cas d'excitation, des signaux pour la détection de la position de l'alésage transversal (14 - 20) à partir des signaux, **caractérisé en ce que** la paroi du fût présente au moins un creux ou une perforation qui loge l'unité d'émission (42 - 48) durablement à l'intérieur du fût du clou intramédullaire (10).

2. Clou intramédullaire selon la revendication 1, **caractérisé en ce qu'**un insert (24) est prévu dans le fût et présente dans sa paroi au moins un creux ou une perforation (34 - 40) qui loge l'unité d'émission (42 - 48).

3. Clou intramédullaire selon la revendication 2, **caractérisé en ce que** l'insert (24) est constitué en forme de douille avec un canal de passage (12) disposé dans la direction longitudinale du clou.

4. Clou intramédullaire selon la revendication 2 ou 3, **caractérisé en ce que** le fût creux présente une dépression avec un talon ou une saillie (22) faisant saillie vers l'intérieur, l'insert inséré dans le fût appuyant dans la direction axiale sur le talon ou la saillie et réalisant une fermeture affleurante avec la paroi intérieure du fût.

5. Clou intramédullaire selon la revendication 2 ou 3, **caractérisé en ce que** l'insert (24) présente des alésages (26 - 32) disposés transversalement à la direction longitudinale et qui sont alignés avec les alésages transversaux (16 - 20) dans le fût.

6. Clou intramédullaire selon une des revendications 2 ou 3, **caractérisé en ce qu'**un élément de contact (50) en forme de douille à l'intérieur de l'insert (24) masque le creux ou la perforation et est connecté à l'équipement d'émission (42 - 48) de façon électriquement conductrice et est isolé électriquement vis-à-vis de l'insert (24).

7. Clou intramédullaire selon la revendication 6, **caractérisé en ce que**, entre l'équipement d'émission (48) et l'insert (24) est disposé un isolateur (52) qui isole l'équipement d'émission (48) vis-à-vis de l'insert (24) et **en ce que** l'équipement d'émission (48) forme une liaison électriquement conductrice avec le fût.

8. Clou intramédullaire selon une des revendications 1 à 7, **caractérisé en ce que** le fût présente un canal (12) creux.

9. Clou intramédullaire selon une des revendications 2 à 8, **caractérisé en ce que** les équipements d'émission (42 - 48) sont disposés respectivement par paires, décalés les uns par rapport aux autres dans la direction axiale.

10. Clou intramédullaire selon une des revendications 2 à 9, **caractérisé en ce que** l'équipement d'émission (42 - 48) présente la forme d'une pastille dont une face frontale est bombée de façon concave adaptée au diamètre intérieur du fût et dont la hauteur correspond essentiellement à la profondeur du creux ou de la perforation (34 - 40).

11. Clou intramédullaire selon une des revendications 1 à 10, **caractérisé en ce que** l'équipement d'émission présente un composant passif ou actif qui émet un signal d'émission en réponse à des signaux d'excitation reçus ou appliqués.

12. Clou intramédullaire selon la revendication 11, **caractérisé en ce que** le composant est constitué en tant que circuit oscillant passif.

13. Clou intramédullaire selon une des revendications 11 ou 12, **caractérisé en ce que** l'équipement d'émission présente deux contacts électriques pour l'application d'un signal d'excitation au moyen d'un conducteur introduit dans le fût.

14. Clou intramédullaire selon la revendication 13, **caractérisé en ce qu'**un des contacts est en liaison électriquement conductrice avec le fût du clou intramédullaire.
